# EUROPEAN PATENT SPECIFICATION

(11) **EP 1 673 057 B1**
(45) Date of publication and mention of the grant of the patent: **29.04.2009**
(21) Application number: 04762914.2
(22) Date of filing: 11.10.2004
(51) Int. Cl.: A61K 8/18

(54) **HAIR GROWTH INHIBITION**
HAARWUCHSHEMMUNG
INHIBITION DE LA POUSSE DE POILS

(30) Priority: 10.10.2003 DK 200301500; 01.12.2003 US 525967 P
(43) Date of publication of application: 28.06.2006
(73) Proprietor: Cosmedical APS, 1103 Copenhagen K (DK)
(72) Inventor: PEDERSEN, Jens, Richard, DK-7100 Vejle (DK); PEDERSEN, Torben, Richard, DK-7120 Vejle Ost (DK)
(74) Representative: HOEIBERG A/S
(86) International application number: PCT/DK2004/000695
(87) International publication number: WO 2005/034892

(56) References cited:
- EP-A- 0 622 069
- EP-A- 0 755 673
- WO-A-00/12663
- WO-A-02/08398
- WO-A-02/22103
- WO-A-96/40854
- WO-A-98/02134
- US-A- 6 117 433
- US-A1- 2003 026 794
- US-B1- 6 375 948
- DATABASE CAPLUS [Online] XP002277142 retrieved from STN Database accession no. 2003:216873 & JP 2003 081793 A (LION CORP.) 19 March 2003 (2003-03-19)

## Description

### Field of invention

The present invention relates to a use of a two-component system for the preparation of a hair growth inhibitor. The present invention concerns a two-component hair growth inhibiting system comprising at least one enzyme.

### Background of invention

To meet the requirement from the public to avoid un-wanted hair on various body parts such as legs, arms, and facial region, a number of hair removing methods have been developed, such as using a shaver, wax-based removal of hairs or the like, or chemical removal agents.

The use of thiol-based depilatory agents, for removal of unwanted body and facial hair is well established in the art (see eg. US 4,941,885; US 4,631,064; US 4,618,344). These agents react by reducing hair's protein disulfide bonds to sulfhydryl anions, thereby allowing easy removal of the weakened hairs when washed or wiped away. The combination of thiol compounds with high pH's results in ionized thiols and increased penetration of a reactant. Similarly, urea or other nitrogen-containing substances have been found to accelerate the action of the thiol-component in hair removal.

Another approach to avoid un-wanted hair is the development of hair growth inhibitors which act on the hair follicle.

Hair grows from the hair follicle. The total number of hair follicles for an adult human is estimated at 5 million with 1 million on the head of which 100,000 alone cover the scalp. ln humans, the only external regions of skin devoid of hair follicles are the palms of the hands and soles of the feet. The basic hair follicle structure remains essentially the same throughout the range of mammalian species with modifications for specialized functions.

The hair follicle can be recognized as a separate entity within the skin with formation and maintenance based on interaction between dermal and epidermal components. The hair follicle is an epithelial structure which undergoes growth cycles throughout adult life. The growth of the hair follicle down into the dermis forming a layered structure with a pigmented shaft is known as the anagen phase whereas a regression phase is known as the catagen phase in which the hair follicle shortens an a part of the hair follicle undergoes programmed cell death known as apoptosis. Also, a hair follicle rest phase has been characterize and named the telogen phase (Seiberg et al. 1997. Dev. Dynamics 208, 553-564. Trypsin-induced follicular papilla apoptosis in delayed hair growth and pigmentation).

Proteolytic enzymes hydrolyze or break down proteins. Proteolytic enzymes are used in hair depilation due to their ability to degrade stem cells and hair follicles. Examples of proteolytic enzymes are papain, chymotrypsin, bromlain and papain.

Topical trypsin treatment of mice following depilation has been shown to induce apoptosis in the follicular papillae which results in delay in hair growth (Seiberg et al. 1997). WO 98/02134 describes the use of a trypsin-containing composition using as pharmaceutical vehicle non-ionic liposomes consisting of glycerol dilaurate, cholesterol-like compounds and fatty acid ethers. Mice are after wax-depilation treated with the composition and hair growth is effectively delayed and apoptosis at the follicular papillae is observed.

In EP 0622069, papain and chymotrypsin are used in a composition for permanent hair depilation. The proteolytic enzymes are stored in powder form in the presence of lactose. In a liquid solution, the enzyme activity is stabilized by the use of dithiotreitol, ethylene glycol, and ethylenediaminotetraacetic acid (EDTA). The solution is buffered to a suitable pH according to the proteolytic enzyme present in the solution.

Immediately before use, the enzyme is dissolved in the liquid. The depilatory activity will be retained for only 7 days when the enzyme-containing solution is stored at 2-6°C and for 4 weeks if kept at -15 to -20°C. The enzyme-containing liquid is supplied by ionophoresis.

Another approach for suppressing hair growth is presented in US 6,375,948 in which plant extracts from the family Juniperus or malts-from cereals are the major active compound. Proteolytic enzymes such as papain, chymotrypsin, bromelain, ficin, pancreatin, pepsin or trypsin may or may not be added in the composition. A number of components usually employed for cosmetics selected from the groups of oils, ethanols, thickeners, emulsifiers, humectants, colors, and perfumes etc. are also included in the composition. The hair growth inhibitor is applied to the skin of mice following shaving of a selected area of the mice. One application per day was repeated for 4 weeks.

WO 97/44005 describes a depilatory preparation that is applied to the skin by spreading. The preparation comprises proteolytic enzymes such as chymotrypsin or papain solubilized in a microemulsion. The microemulsion contains an inert organic agent and droplets of water in which the proteolytic enzymes are present. The water droplets are dispersed in the organic phase by use of lecithin as a surfactant. The organic phase is kept separate from the enzyme-containing water until use. Just prior to use, the enzyme solution and the organic phase are mixed by gentle shaking to product the active depilatory preparation. The depilatory action persists for 1-3 days if stored at 2-6°C.

### Summary of invention

In one aspect, the invention relates to a use of a two-component system comprising

A first component comprising
a first composition comprising
   at least one enzyme, said enzyme being dissolved in as solvent and
   a water activity reducing agent, wherein said water activity reducing agent constitutes at least 50 percent by weight of the composition, a second component comprising
a second composition, comprising
   at least one enzyme-activating agent and/or
   at least one anti-inflammatory, penetration-promoting agent and/or
   a preservative agent
   for the preparation of a hair growth inhibitor.

By introducing a water activity reducing agent in the composition it is possible to provide a composition capable of prolonged storage without any substantial degradation of the enzyme component of the composition.

### Description of drawings

Figure 1
   shows a schematic presentation of a hair follicle, where in the main differentiated layers in a mature anagen hair follicle are shown.
   The hair follicle penetrates the dermal layer of the skin composed of fibroblast cells and collagen connective tissue interspersed with blood vessels, sweat glands and sensory nerves. The bulb region sits just above the subcutaneous (adipose fat) tissue layer. (Figure from www.keratin.com).
Figure 2
   show an assay for proteolytic activity.
   Hair inhibiting products are applied to a petridish containing casein.
   Application 1: Commercial hair inhibiting product, containing papain (Surgi-Hair Stop, Ardell international). Product stored at room temperature.
   Application 2: Commercial hair inhibiting product, containing papain. Product stored at room temperature (GiGi No More Hair (American International industries). Application 3: Composition according to this invention, comprising trypsin. Stored at room temperature for 5 months and 24 days prior to application.
Figure 3 Photo of lower leg.
   Top: Photo taken before first treatment.
   Bottom: Photo taken before treatment number 4 which corresponds to 6 weeks after treatment number 3.
   Treatment 1: 14 May 2003
   Treatment 2: 24 June 2003
   Treatment 3: 5 August 2003
   Treatment 4:16 September 2003
Figure 4. Photo of back.
   Top: Photo taken before first treatment.
   Bottom: Photo taken before treatment number 3 which corresponds to 6 weeks after treatment number 2.
   Treatment 1: 2 July 2003
   Treatment 2:20 August 2003
   Treatment 3: 1 October 2003.
Figure 5. Photo of armpit (Axilla).
   Top: Photo taken before first treatment.
   Bottom: Photo taken before treatment number 4 which corresponds to 9 weeks after treatment number 3.
   Treatment 1:12 March 2003
   Treatment 2: 7 May 2003
   Treatment 3: 3 july 2003
   Treatment 4: 11 September 2003.
Figure 6. Photo of upper lip.
   Top: Photo taken before first treatment.
   Bottom: Photo taken before treatment number 6 which corresponds to 4 weeks after treatment number 5.
   Treatment 1:1April 2003
   Treatment 2: 29 April 2003
   Treatment 3: 27 May 2003
   Treatment 4:16 June 2003
   Treatment 5: 14 July 2003
   Treatment 6: 12 August 2003

### Detailed description of the invention

### Definitions

### Enzyme activity:

Enzyme activity determined by the quantity of substrate transformed or product formed per unit time. The reaction rate that is measured depends on a number of experimental conditions such as temperature, pH, ionic strength, and the presence or absence of inhibitors or activators. It is only under the conditions specified in the prescribed assay procedure that enzyme units are defined.

The biological biopolymeric substrates (proteins, polysaccharides, bacteria, oil emulsions) often have a molecular complexity and the fact that the potential number of bonds susceptible to hydrolysis is not known and frequently varies over the time course of the assay makes a Good Standardization Practice necessary. The International Commission on Enzymes F.I.P. calculates the potency in terms of the F.I.P. -standard using a unitage expressed in "International F.I.P. -units.

The activity of a given enzyme is thus influenced by a number of parameters such as chemical and physical conditions as described above. The enzyme activity is also influenced by the action the enzyme itself, a process known as self degradation, self destruction or self digestion. Loss of enzyme activity may thus be due to any of the reasons listed.

### Water activity:

Water activity is defined as the qualitative measurement of water's energy state in a given system. The principles from thermodynamics and physical-chemical properties form the basis for this definition. The water activity of a given material depends on the types of interactions the material forms with other components in the composition such as ionic binding, dipolar forces, Van der Waals force, hydroxyl groups in sugars, carbonyl and amino groups in proteins etc. In contrast, moisture is the quantity of water in a sample measured as dry basis or moist basis which depends on the amount of material present. Consequently, water activity provides information on e.g. water migration, chemical and biochemical stability, physical properties, and shelf-life which are not provided by measurement of moisture.

### Water activity reducing agents:

Water activity reducing agents serve to reduce the water activity in a given composition. Water reducing agents are commonly used in foods where the agents serve to improve shelf-life of foods and to reduce the growth of pathogenic bacteria such as *Clostridium botulinum* in foods. Polyols such as propylene glycol, glycerol (glycerine), sorbitol, mannitol, are added to foods to provide sugar crystallization modification, water binding, or cryoprotection. Propylene glycol is primarily used as a solvent for colors, antioxidants and flavours but also serves to reduce water activity. Glycerol in foods is primarily used as plasticizer or water activity lowering compound.

### Use

The present invention relates to a use of a system for the preparation of a hair growth inhibitor. The hair growth inhibitor comprises at least one enzyme, at least one solvent, and at least one water activity reducing agent as described herein. In a preferred embodiment of the invention the enzyme, solvent and water activity reducing agent is mixed.

In a further preferred embodiment the water activity reducing agent constitutes at least 50 percent by weight of the composition, such as at least 70 %, such as at least 75%, such as at least 80%, such as at least 90%, such as at least 95 %, such as at least 98%, such as at least 99 % by weight of the composition.

### Composition

The problem with prior art compositions is among others that proteolytic enzymes when stored in a solvent are degraded unless stored in a freezer. Consequently, proteolytic enzymes are often stored in powder form at temperatures below 2-6°C or in the freezer. Furthermore, some prior art compositions need to be mixed immediately before use in order to be effective. Thus, the prior art compositions are not practical to use, in particular not practical to use by the consumer herself at home.

The present invention offers a composition that may be stored at room temperature for a prolonged period of time without degrading the enzymes, thereby facilitating the use of the composition. Accordingly, the composition according to the invention may be used both by specialists and by the normal consumer, since the product is easy to apply and requires no special storage conditions.

The advantages by the present invention have been obtained by incorporating into the composition an agent capable of reducing the water activity of the composition, whereby self-degradation of the enzyme is reduced or even absent. It is shown that when glycerol constitutes about 67 percent of the composition according to the invention the water activity is reduced to about 0.7 as measured as described herein. Furthermore, in a preferred embodiment the water activity is reduced to about 0.4 when glycerol constitutes about 83 percent by weight of the composition. Moreover, in a preferred embodiment the water activity is reduced to about 0.27 when glycerol constitutes about 92 percent by weight of the composition. In an even more preferred embodiment the water activity is about 0.25 when glycerol constitutes about 96 percent by weight of the composition. In an even more preferred embodiment the water activity is below 0.07 when glycerol constitutes 96.5 percent of the composition.

The degradation reduced by the water activity reducing agent is primarily the self-degradation known to occur in enzymes dissolved in a solvent.

The water activity reducing agent according to the invention may be any agent capable of reducing the water activity, in particular an agent capable of reducing the water activity to a value below 0.3 when measured according to the assay as described herein.

In a preferred embodiment the water activity reducing agent may be selected from the propylene glycol, glycerine, sorbitol, saccharose, and saline, or a combination thereof. In a more preferred embodiment the water activity reducing agent is glycerine

The water activity reducing agent is present in an amount sufficient to provide the water activity reducing effect. Accordingly, it is preferred that the water activity reducing agent constitutes at least 60 percent by weight of the composition, such as at least 70 percent by weight of the composition, at least 80 percent by weight of the composition, at least 85 percent by weight of the composition, at least 90 percent by weight of the composition, at least 92 percent by weight of the composition, at least 95 percent by weight of the composition.

### Storage time

The water activity reducing agent as described above is able to prolong the storage time of proteolytic enzyme, as the reduction of water activity will prevent self-destruction of the proteolytic enzyme. It is desirable for the consumer herself that the enzyme is active upon storage for a long period of time. In a preferred embodiment of the invention the storage time of the proteolytic enzyme-containing composition at room temperature is at least 12 months, such as at least 9 months, at least 6 months, at least 3 months, at least 2 months, at least 1 month, at least 3 weeks.

### Proteolytic enzyme

The composition according to the invention comprises at least one enzyme, wherein the enzyme is present in order to hydrolyze or break down proteins in the hair follicle, in particular in a hair follicle wherein the hair has been removed.

The enzyme of the composition may be any suitable enzyme, it is however preferred that the enzyme is a proteolytic enzyme.

In one embodiment of the invention the enzyme is selected from the group of serine proteases, such as trypsin, chymotrypsin, subtilisin or elastase. In another embodiment the enzyme is selected from the group of cysteine proteases, such as bromelain.

In a preferred embodiment the enzyme is selected from the group of enzymes consisting of trypsin, chymotrypsin, papain, tiromelain. In a more preferred embodiment the at least one enzyme is an enzyme selected from the group of enzymes consisting of trypsin and chymotrypsin, and even more preferred the at least one enzyme is trypsin.

In a further preferred embodiment the enzyme is selected from the group of enzymes consisting of trypsin, bromelain or papain. Also preferred is the selection of at least one enzyme from bromelain or papain.

It is preferred that the enzyme constitutes at least 1.0 percent by weight of the composition, such as at least 1.5 percent by weight of the composition, such as at least 2.0 percent by weight of the composition, such as at least 2.5 percent by weight of the composition, such as at least 3.0 percent by weight of the composition.

Furthermore, it is preferred that the enzyme constitutes at most 10 percent by weight of the composition. More preferably the enzyme constitutes at most 7.5 percent by weight of the composition, such as at most 6 percent by weight of the composition, such as at most 5 percent by weight of the composition, such as at most 4 percent by weight of the composition.

In a more preferred embodiment the enzyme constitutes from 2.0 to 3.0 percent by weight of the composition, such as preferably about 2.5 percent by weight of the composition, in particular when the enzyme is trypsin.

In another preferred embodiment the enzyme as described above has an activity of at least 100 F.I.P. units per gram of the composition, such as at least 150 F.I.P. units per gram of the composition, such as at least 175 F.I.P. units per gram of the composition, such as at least 200 F.I.P. units per gram of the composition, such as at least 225 F.I.P. units per gram of the composition, such as at least 250 F.I.P. units per gram of the composition.

Furthermore, it is preferred that the enzyme has an activity of at most 350 F.I.P. units per gram of the composition. More preferably the enzyme has an activity of at most 300 F.I.P. units per gram of the composition, such as 275 F.I.P. units per gram of the composition, 250 F.I.P. units per gram of the composition, such as 225 F.I.P. units per gram of the composition.

In a more preferred embodiment the enzyme has an activity from 150 to 250 F.I.P. units per gram of the composition, such as preferably about 200 F.I.P. units per gram of the composition, in particular when the enzyme is trypsin.

### Solvent

The enzyme of the composition according to the invention is dissolved in a solvent. The solvent may be any suitable solvent. In a preferred embodiment the solvent is water.

In order to reduce the water activity of the composition as much as possible it is desirable to only include, as little as possible of the solvent. Accordingly, it is preferred that the solvent and the enzyme constitutes at the most 20 percent by weight of the composition. More preferably the solvent and the enzyme constitute at the most 15 percent by weight of the composition, such as at the most 10 percent by weight of the composition, such as at the most 5 percent by weight of the composition, such as at the most 3 percent by weight of the composition. In particular, the solvent and the enzyme constitute about 2.9 percent per weight of the composition.

### Polymer

The solvent of the composition according to the invention may be cross-bound by a polymer. The polymer may be any suitable polymer. In a preferred embodiment the polymer comprises acrylic acid monomers. In an even more preferred embodiment the polymer is an acrylic acid polymer. More preferably, the polymer is selected from carbomers such carboxymethylene resins, polyacrylic acid, C10-C30 alkyl propenoate, polymer with propenoic acid, butenoic acid and/or alkyl propenoates, product with propenyl sucrose ether or propenyl 2,2-dihydroxymethyl-1.3-propanediol. In another preferred embodiment the polymer is a combination of the polymers described. In an even more preferred embodiment the polymer is Carbopol® 940 NF polymer(Noveon, Ohio).

In order to cross-bind the solvent of the composition as effectively as desirable, it is preferred that the polymer constitutes at least 0.05 percent by weight of the composition, such as at least 0.1 percent by weight of the composition, at least 0.15 percent by weight of the composition, at least 0.2 percent by weight of the composition, at least 0.25 percent by weight of the composition, at least 0.275. percent by weight of the composition, at least 0.3 percent by weight of the composition.

Furthermore, it is preferred that the polymer constitutes at most 3 percent by weight of the composition. More preferably, the polymer constitutes such as at most 2 percent by weight of the composition, such as at most 1 percent by weight of the composition, such as at most 0.5 percent by weight of the composition, such as at most 0.4 percent by weight of the composition.

In a more preferred embodiment the polymer constitutes from 0.15 to 0.25 percent by weight of the composition, such as preferably about 0.2 percent by weight of the composition, in particular when the polymer is Carbopol® 940 NF polymer (Noveon, Ohio).

### Neutralization

The polymer according to the invention may be neutralized by a neutralizing agent if relevant. The neutralizing agent may be any suitable neutralizing agent, capable of neutralizing the polymer in question. In a preferred embodiment the neutralizing agent is selected from triethanolamine or diisopropanolamine. In a further preferred embodiment the neutralizing agent is diisopropanolamine.

In order to neutralize the polymer of the composition as effectively as desirable, it is preferred that the neutralizing agent constitutes at least 0.1 percent by weight of the composition, such as at least 0.2 percent by weight of the composition, at least 0.3 percent by weight of the composition, at least 0.35 percent by weight of the composition, at least 0.4 percent by weight of the composition.

Furthermore, it is preferred that the neutralizing agent constitutes at most 2 percent by weight of the composition. More preferably, the polymer constitutes such as at most 1 percent by weight of the composition, at most 0.5 percent by weight of the composition, at most 0.45 percent by weight of the composition.

In a more preferred embodiment the neutralizing agent constitutes from 0.35 to 0.45 percent by weight of the composition, such as preferably about 0.4 percent by weight of the composition, in particular when the neutralizing agent is diisopropanolamine.

### Two-component system

The ready to use two-component system according to the invention consists of two components acting in combination as a hair growth inhibitor. It is desirable that the two components are not in contact with each other prior to use in order to avoid self-destruction of the proteolytic enzymes. The two components of the system are stored in two separate compartments. In a further preferred embodiment of the invention the two components may be placed in two separate tubes with a screw cap, two containers with a screw cap, two closed flasks or the like.

In a preferred embodiment the first component consists of the composition as described above containing at least one proteolytic enzyme, a solvent, a water activity reducing agent, and optionally a polymer, and a neutralizing agent. Preferably, the composition of the first component of the system is in the form of a creme, a gel or a paste or the like.

In a further preferred embodiment the second composition of the second component of the system according to the invention consists of at least one enzyme-activating agent and/or at least one anti-inflammatory, penetration promoting agent and/or a preservative agent.

The enzyme-activating agent of the second composition of the second component of the system activates the proteolytic enzymes of the first composition of the first component of the system. Preferably, the enzyme-activating agent is a solvent. The solvent may be any of the solvents as described above. Even more preferable, the enzyme-activating agent is water.

It is preferred that the enzyme-activating agent constitutes at least 15 percent by weight of the second composition of the system, such as at least 30 percent by weight, such as at least 40 percent by weight, such as at least 50 percent by weight, such as at least 60 percent by weight, such as at least 70 percent by weight, such as at least 80 percent by weight, such as at least 90 percent by weight, such as at least 95 percent by weight.

Furthermore, it is preferred that the enzyme-activating agent constitutes at most 98 percent of the second composition of the second component of the system, such as at most 97 percent of the second composition of the second component of the system, such as at most 96 percent of the second composition of the second component of the system, such as at most 95 percent of the second composition of the second component of the system.

In a more preferred embodiment the water activating agent constitutes from 90 to 97 percent of the second composition of the second component of the system, such as about 97 percent of the second composition of the system, in particular when the enzyme-activating agent is water.

In order to promote the penetration of the composition into the hair follicle and to reduce potential inflammation brought about by the composition, a preferred embodiment according to the invention is to include, at least one penetration-promoting, anti-inflammatory agent. In a further preferred embodiment the penetration-promoting, anfi-inflammatory agent is selected from the group of salicylates, and even more preferred the penetration-promoting, anti-inflammatory agent is diethylamine salicylate.

It is preferred that the penetration-promoting, anti-inflammatory agent constitutes at least 0.5 percent by weight of the second composition of the system, such as at least 1 percent by weight, such as at least 1.5 percent by weight, such as at least 2 percent by weight of the second composition of the second component of the system.

Furthermore, it is preferred that the penetration-promoting, anti-inflammatory agent constitutes at most 5 percent of the second composition of the second component of the system, such as at most 4 percent of the second composition of the second component of the system, such as at most 3 percent of the second composition of the second component of the system.

In a more preferred embodiment the penetration-promoting, anti-inflammatory agent constitutes from 1.5 to 3 percent of the second composition of the second component of the system, such as about 2 percent of the second composition of the system, in particular when the penetration-promoting, anti-inflammatory agent is diethylamine salicylate.

In order to avoid bacterial growth and the like of the second composition of the second component of the system, a preferred embodiment is to include at least one preservative agent, and even more preferred to Include sodium benzoate.

It is preferred that the preservative agent constitutes at least 0.1 percent by weight of the second composition of the system, such as at least 0.3 percent by weight, such as at least 0.5 percent by weight, such as at least 0.8 percent by weight, such as at least 0.9 percent by weight of the second composition of the second component of the system

Furthermore, it is preferred that the preservative agent constitutes at most 3 percent by weight of the second composition of the second component of the system, such as at most 2 percent by weight, such as at most 1.5 percent by weight, such as at most 1.25 percent by weight, such as at most 1.5 percent by weight of the second composition of the second component of the system.

In a more preferred embodiment the preservative agent constitutes from 0.5 to 1.5 percent of the second composition of the second component of the system, such as about 1 percent of the second composition of the system, in particular when the preservative agent is sodium benzoate.

### Examples

### Reference Example 1

### Formulation without polymer.

| Component I: | |
|---|---|
| Agent: | Percent by weight of composition |
| Water | 0.5 |
| Enzyme (Trypsin) | 2.4 |
| Glycerol | 97.1 |

### Reference Example 2

Example of alternative formulation, using chymotrypsin or bromelain.

| Component I: | |
|---|---|
| Agent: | Percent by weight of composition |
| Water | 0.5 |
| Enzyme (Chymotrypsin or bromelain) | 2.4 |
| Glycerol | 97.1 |

### Example 3

Example of two component system and formulation.

| Component I: | |
|---|---|
| Agent: | Percent by weight of composition |
| Water | 0.5 |
| Enzyme (Trypsin) | 2.4 |
| Glycerol | 96.5 |
| Neutralizing agent (Diisopropanolamin) | 0.4 |
| Polymer (Carbomer) | 0.2 |
| | |

| Component II: | |
|---|---|
| Agent: | Percent by weight of composition |
| Water | 97.4 |
| Polymer (Carbomer) | 0.2 |
| Neutralizing agent (Diisopropanolamine) | 0.4 |
| Penetration-promoting, antiinflammatory | |
| agent (Diethylamine salicylate) | 2.0 |

### Example 4

Alternative formulation of two-component system.

| Component I: | |
|---|---|
| Agent | Percent by weight of composition |
| | |
| Water | 0.5 |
| Enzyme (Trypsin) | 2.4 |
| Glycerol | 96.5 |
| Neutralizing agent (Diisopropanolamin) | 0.4 |
| Polymer (Carbomer) | 0.2 |
| | |

| Component II: | |
|---|---|
| Agent: | Percent by weight of composition |
| | |
| Water | 98,0 |
| Penetration-promoting, antiinflammatory | |
| Agent (Diethylamine salicylate) | 2.0 |

### Example 5

Water activity (wa) measurements of the components of the system.

| Components of the system | Water % | wa |
|---|---|---|
| Component I (enzyme + glycerine) | 0.5 | 0.069 |
| Component II (enzyme activation) | 94.5 | 0.964 |
| 1:1 ratio Component I and II | 49.75 | 0.915 |
| Glycerin with water | 4.14 | 0.246 |
| Glycerin with water | 8.28 | 0.275 |
| Glycerin with water | 16.56 | 0.420 |
| Glycerin with water | 33.12 | 0.657 |

Water percentage was measured using SCALTEC SMO 01 apparatus (SCALTEC INSTRUMENTS).

Wa value measured using TESTO-400 (TESTO AG).

Water % is water in percent by weight of composition.

### Example 6

Effect on hair growth inhibition in vivo, using the composition on various body parts.

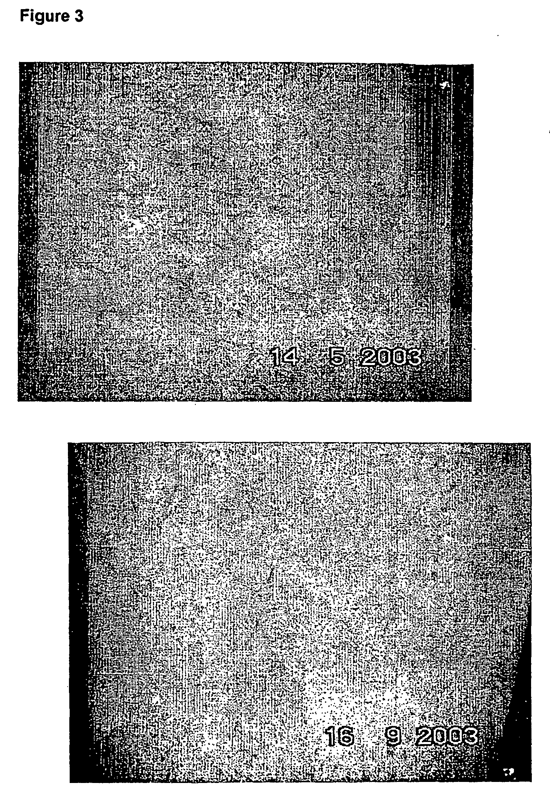

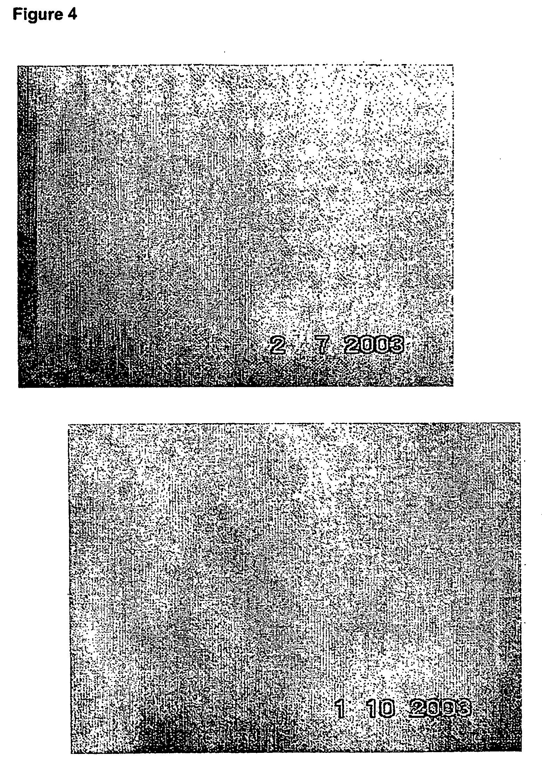

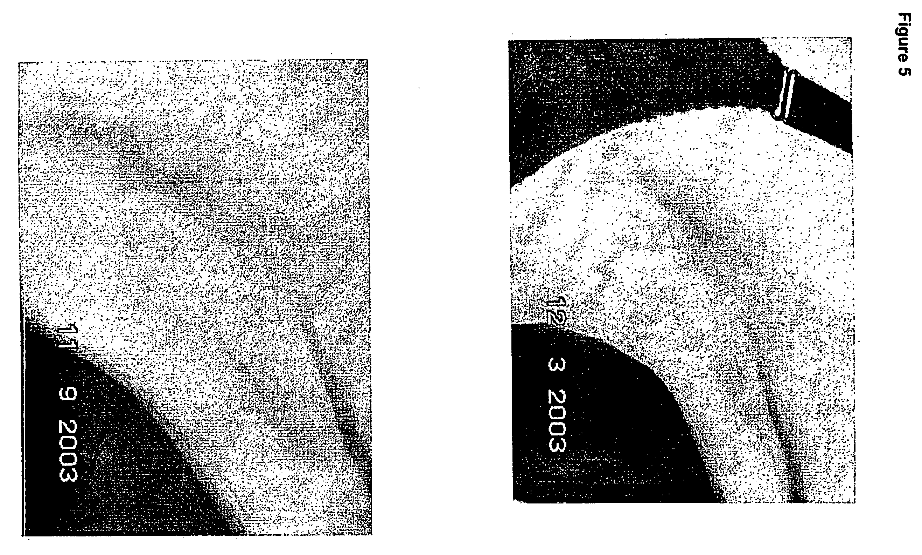

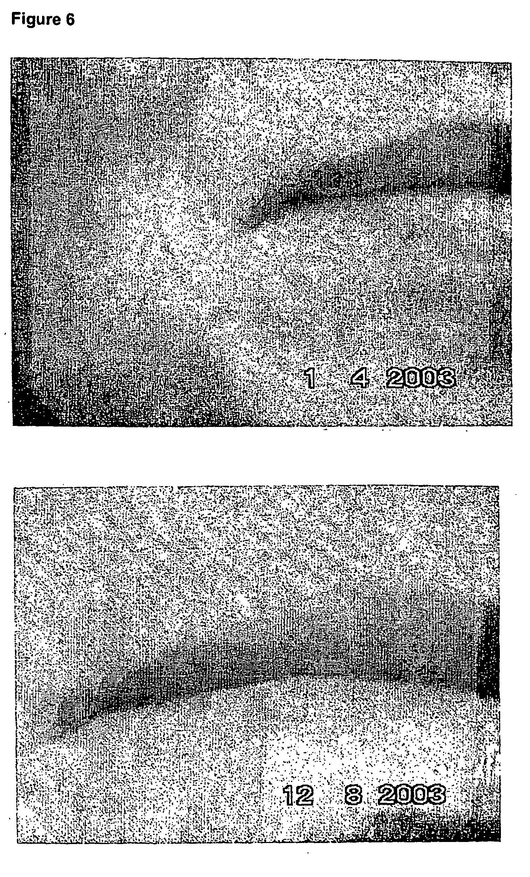

The hair growth inhibitor was applied to lower leg, back, armpit, and upper lip of humans at defined time intervals. The hair growth inhibitor was applied as a thin homogenous layer, using 1 ml per 20 cm² skin. Photographs were taken prior to the beginning of treatment and at various time points during the repeated treatments, the result of which are shown in Figures 3, 4, 5 and 6.

### Example 7

### Measurement of activity and effect of storage in vitro:

### Casein used as substrate for proteolytic degradation.

20 ml Ca-caseinate/agar solution is distribute in a petri dish (Ca-caseinate solution: 0.5 g Ca-caseinate, 1 g agar, 98.5 g 0.9 % NaCl solution).10 µ of sample (diluted 1:10 in water) is loaded into 3 mm application hole. Petri dish incubated at 37°C for 4 hrs.

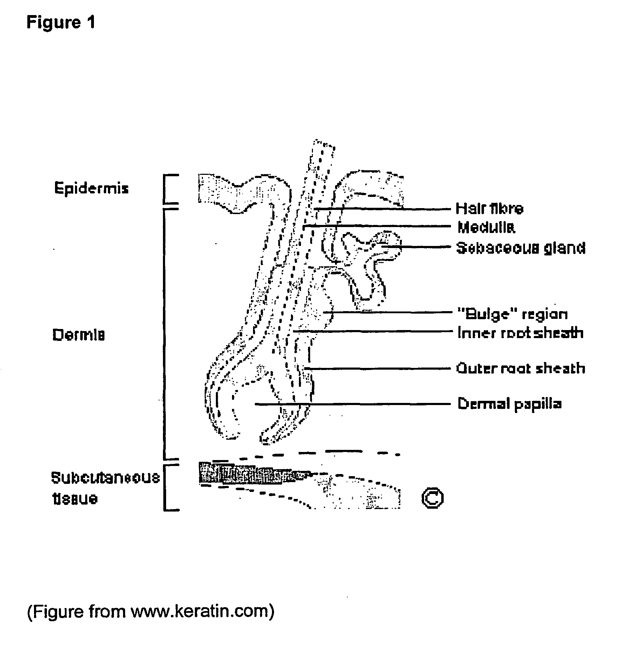

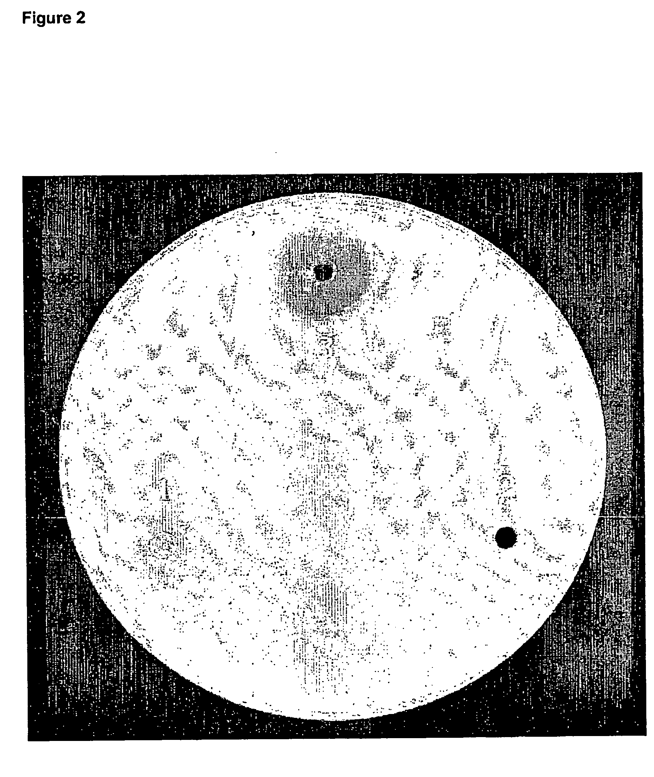

Figure 2 shows the activity of the composition upon storage for about 6 months, using the activity measurement described above. The activity of the composition is compared to the activity of alternative commercial products.

Alternative commercial products show minimal activity, whereas the composition according to the invention displays high activity upon prolonged storage at room temperature.

Application 1: Commercial hair inhibiting product, containing papain (Surgi-Hair Stop, Ardell International). Product stored at room temperature. Application 2: Commercial hair inhibiting product, containing papain. Product stored at room temperature (GiGi No More Hair (American International Industries). Application 3: Composition according to this invention, comprising trypsin. Stored at room temperature for 5 months and 24 days prior to application.

### Reference Example 8

The enzyme of the composition is dissolved in water. Enzyme and water constitutes 2.9 percent by weight of the composition. Water constitutes 2.4 percent by weight of the composition and enzyme constitutes 0.5 percent by weight of the composition.

## Claims

1. Use of a two-component system comprising
A first component comprising
a first composition comprising
at least one enzyme, said enzyme being dissolved in a solvent, and
a water activity reducing agent, wherein said water activity reducing agent constitutes at least 50 percent by weight of the composition,
a second component comprising
a second composition, comprising
at least one enzyme-activating agent and/or
at least one anti-inflammatory, penetration-promoting agent and/or
a preservative agent
for the preparation of a hair growth inhibitor.

2. The use of the system according to claim 1, wherein the at least one enzyme of the first composition is a proteolytic enzyme.

3. The use of the system according to claim 2, wherein the at least one enzyme of the first composition is an enzyme selected from the group of enzymes consisting of trypsin, chymotrypsin, papain, bromelain.

4. The use of the system according to claim 1, wherein the solvent of the first composition is water.

5. The use of the system according to any of the preceding claims, wherein the water activity reducing agent of the first composition is selected from glycerine, sorbitol, saccharose, saline.

6. The use of the system according to any of the preceding claims, wherein the water activity reducing agent of the first composition constitutes at least 60 percent by weight of the composition, such as at least 70 percent by weight of the composition, at least 80 percent by weight of the composition at least 85 percent by weight of the composition, at least 90 percent by weight of the composition, at least 92 percent by weight of the composition, at least 95 percent by weight of the composition, at least 98 percent by weight of the composition

7. The use of the system according to claim 1, wherein said first composition further comprises a polymer.

8. The use of the system according to claim 7, wherein the polymer comprises acrylic acid monomers.

9. The use of the system according to claims 7, wherein the polymer constitutes 0.2 percent by weight of the first composition.

10. The use of the system according to claims 7-9, wherein the polymer of the first composition constitutes at most 1.5 percent by weight of the composition, such as at most 1 percent by weight of the composition, at most 0.5 percent by weight of the composition at most 0.3 percent by weight of the composition, at most 0.275 percent by weight of the composition, at most 0.25 percent by weight of the composition, at most 0.2 percent by weight of the composition.

11. The use of the system according to any of the preceding claims, wherein the enzyme of the first composition constitutes at least 7.5 percent by weight of the composition, such as at least 6 percent by weight of the composition, at least 5 percent by weight of the composition at least 4 percent by weight of the composition, at least 3 percent by weight of the composition, at least 2.75 percent by weight of the composition, at least 2.5 percent by weight of the composition.

12. The use of the system according to any of the preceding claims, said first composition further comprising an agent capable of neutralising the polymer.

13. The use of the system according to claim 12, wherein the agent is diisopropanolamin.

14. The use of the system according to any of the preceding claims, wherein the first composition is in the form of a creme, a paste, a gel or a liquid.

15. The use of the system according to claim 1, wherein the two compositions of the two components of the system are in separate compartments.

16. The use of the system according to claim 1, wherein the second composition of the second component of the system comprises the at least one enzyme-activating agent consisting essentially of a solvent.

17. The use of the system according to claim 1, wherein the second composition of the second component of the system comprises the at least one enzyme-activating agent consisting essentially of water.

18. The use of the system according to claim 1, wherein the second composition of the second component of the system further comprises at least one penetration-promoting, anti-inflammatory agent.

19. The use of the system according to claim 1, wherein the second composition of the second component of the system further comprises the at least one penetration-promoting, anti-inflammatory agent selected from a group of salicylates.

20. The use of the system according to claim 1, wherein the second composition of the second component of the system further comprises at least one preservative agent.

21. The use of the system according to any of the claims 1-8, wherein the at least one enzyme-activating agent of the second composition of the second component of the system constitutes 95 percent by weight of the second component.

22. The use of the system according to any of the claims 1-8, wherein the at least one penetration-promoting, anti-inflammatory agent of the second composition of the second component of the system constitutes 2 percent by weight of said second composition of the second component of the system.

23. The use of the system according to any of the claims 1-8, wherein the at least one preservative agent of the second composition of the second component of the system constitutes 1 percent by weight of said second composition of the second component of the system.

## Patentansprüche

1. Verwendung eines Zweikomponentensystems, umfassend:
Eine erste Komponente, umfassend:
eine erste Zusammensetzung, umfassend:
mindestens ein Enzym, wobei das Enzym in einem Lösungsmittel gelöst vorliegt, und
ein die Wasseraktivität reduzierendes Mittel, worin das die Wasseraktivität reduzierende Mittel mindestens 50 Gew.-% der Zusammensetzung bildet,
eine zweite Komponente, umfassend:
eine zweite Zusammensetzung, umfassend:
mindestens ein ein Enzym aktivierendes Mittel und/oder mindestens ein anti-entzündliches, eine Durchdringung förderndes
Mittel und/oder
ein Konservierungsmittel,
für die Herstellung eines Haarwachstumshemmstoffs.

2. Verwendung des Systems nach Anspruch 1, worin das mindestens eine Enzym der ersten Zusammensetzung ein proteolytisches Enzym ist.

3. Verwendung des Systems nach Anspruch 2, worin das mindestens eine Enzym der ersten Zusammensetzung ein Enzym ist ausgewählt aus der Gruppe von Enzymen bestehend aus Trypsin, Chymotrypsin, Papain, Bromelain.

4. Verwendung des Systems nach Anspruch 1, worin das Lösungsmittel der ersten Zusammensetzung Wasser ist.

5. Verwendung des Systems nach einem der vorstehenden Ansprüche, worin das die Wasseraktivität reduzierende Mittel der ersten Zusammensetzung ausgewählt ist unter Glycerin, Sorbitol, Saccharose, Salzlösung.

6. Verwendung des Systems nach einem der vorstehenden Ansprüche, worin das die Wasseraktivität reduzierende Mittel der ersten Zusammensetzung mindestens 60 Gew.-% der Zusammensetzung bildet, wie beispielsweise mindestens 70 Gew.-% der Zusammensetzung, mindestens 80 Gew.-% der Zusammensetzung, mindestens 85 Gew.-% der Zusammensetzung, mindestens 90 Gew.-% der Zusammensetzung, mindestens 92 Gew.-% der Zusammensetzung, mindestens 95 Gew.-% der Zusammensetzung, mindestens 98 Gew.-% der Zusammensetzung.

7. Verwendung des Systems nach Anspruch 1, worin die erste Zusammensetzung weiterhin ein Polymer umfasst.

8. Verwendung des Systems nach Anspruch 7, worin das Polymer Acrylsäuremonomere umfasst.

9. Verwendung des Systems nach Anspruch 7, worin das Polymer 0,2 Gew.-% der ersten Zusammensetzung bildet.

10. Verwendung des Systems nach Ansprüchen 7-9, worin das Polymer der ersten Zusammensetzung maximal 1,5 Gew.-% der Zusammensetzung bildet, wie beispielsweise maximal 1 Gew.-% der Zusammensetzung, maximal 0,5 Gew.-% der Zusammensetzung, maximal 0,3 Gew.-% der Zusammensetzung, maximal 0,275 Gew.-% der Zusammensetzung, maximal 0,25 Gew.-% der Zusammensetzung, maximal 0,2 Gew.-% der Zusammensetzung.

11. Verwendung des Systems nach einem der vorstehenden Ansprüche, worin das Enzym der ersten Zusammensetzung mindestens 7,5 Gew.-% der Zusammensetzung bildet, wie beispielsweise mindestens 6 Gew.-% der Zusammensetzung, mindestens 5 Gew.-% der Zusammensetzung, mindestens 4 Gew.-% der Zusammensetzung, mindestens 3 Gew.-% der Zusammensetzung, mindestens 2,75 Gew.-% der Zusammensetzung, mindestens 2,5 Gew.-% der Zusammensetzung.

12. Verwendung des Systems nach einem der vorstehenden Ansprüche, wobei die erste Zusammensetzung weiterhin ein Mittel umfasst, das das Polymer neutralisieren kann.

13. Verwendung des Systems nach Anspruch 12, worin das Mittel Diisopropanolamin ist.

14. Verwendung des Systems nach einem der vorstehenden Ansprüche, worin die erste Zusammensetzung in der Form einer Creme, einer Paste, eines Gels oder einer Flüssigkeit vorliegt.

15. Verwendung des Systems nach Anspruch 1, worin die zwei Zusammensetzungen der zwei Komponenten des Systems in getrennten Kompartimenten vorliegen.

16. Verwendung des Systems nach Anspruch 1, worin die zweite Zusammensetzung der zweiten Komponente des Systems das mindestens eine ein Enzym aktivierende Mittel umfasst, das im Wesentlichen aus einem Lösungsmittel besteht.

17. Verwendung des Systems nach Anspruch 1, worin die zweite Zusammensetzung der zweiten Komponente des Systems das mindestens eine ein Enzym aktivierende Mittel umfasst, das im Wesentlichen aus Wasser besteht.

18. Verwendung des Systems nach Anspruch 1, worin die zweite Zusammensetzung der zweiten Komponente des Systems weiterhin mindestens ein eine Durchdringung förderndes, anti-entzündliches Mittel umfasst.

19. Verwendung des Systems nach Anspruch 1, worin die zweite Zusammensetzung der zweiten Komponente des Systems weiterhin mindestens ein eine Durchdringung förderndes, anti-entzündliches Mittel umfasst, das aus einer Gruppe von Salicylaten ausgewählt ist.

20. Verwendung des Systems nach Anspruch 1, worin die zweite Zusammensetzung der zweiten Komponente des Systems weiterhin mindestens ein Konservierungsmittel umfasst.

21. Verwendung des Systems nach einem der Ansprüche 1-8, worin das mindestens eine ein Enzym aktivierende Mittel der zweiten Zusammensetzung der zweiten Komponente des Systems 95 Gew.-% der zweiten Komponente bildet.

22. Verwendung des Systems nach einem der Ansprüche 1-8, worin das mindestens eine eine Durchdringung fördernde, anti-entzündliche Mittel der zweiten Zusammensetzung der zweiten Komponente des Systems 2 Gew.-% der zweiten Zusammensetzung der zweiten Komponente des Systems bildet.

23. Verwendung des Systems nach einem der Ansprüche 1-8, worin das mindestens eine Konservierungsmittel der zweiten Zusammensetzung der zweiten Komponente des Systems 1 Gew.-% der zweiten Zusammensetzung der zweiten Komponente des Systems bildet.

## Revendications

1. Utilisation d'un système à deux composants comprenant
un premier composant comprenant
une première composition comprenant
au moins une enzyme, ladite enzyme étant dissoute dans un solvant, et
un agent réduisant l'activité de l'eau, lequel agent réduisant l'activité de l'eau constituant au moins 50 % en poids de la composition,
un deuxième composant comprenant
une deuxième composition comprenant
au moins un agent activateur d'enzyme et / ou au moins un agent anti-inflammatoire, favorisant la pénétration, et / ou
un agent conservateur
pour la préparation d'un inhibiteur de la pousse des poils.

2. Utilisation du système selon la revendication 1, dans laquelle l'au moins une enzyme de la première composition est une enzyme protéolytique.

3. Utilisation du système selon la revendication 2, dans laquelle l'au moins une enzyme de la première composition est une enzyme choisie dans le groupe d'enzymes constitué par la trypsine, la chymotrypsine, la papaïne, la bromélaïne.

4. Utilisation du système selon la revendication 1, dans laquelle le solvant de la première composition est l'eau.

5. Utilisation du système selon l'une quelconque des revendications précédentes, dans laquelle l'agent réduisant l'activité de l'eau de la première composition est choisi parmi la glycérine, le sorbitol, le saccharose, la solution salée.

6. Utilisation du système selon l'une quelconque des revendications précédentes, dans laquelle l'agent réduisant l'activité de l'eau de la première composition représente au moins 60 % en poids de la composition, par exemple au moins 70 % en poids de la composition, au moins 80 % en poids de la composition, au moins 85 % en poids de la composition, au moins 90 % en poids de la composition, au moins 92 % en poids de la composition, au moins 95 % en poids de la composition, au moins 98 % en poids de la composition.

7. Utilisation du système selon la revendication 1, dans laquelle ladite première composition comprend en outre un polymère.

8. Utilisation du système selon la revendication 7, dans laquelle le polymère comprend des monomères d'acide acrylique.

9. Utilisation du système selon la revendication 7, dans laquelle le polymère représente 0,2 % en poids de la première composition.

10. Utilisation du système selon les revendications 7 à 9, dans laquelle le polymère de la première composition représente au plus 1,5 % en poids de la composition, par exemple au plus 1 % en poids de la composition, au plus 0,5 % en poids de la composition, au plus 0,3 % en poids de la composition, au plus 0,275 % en poids de la composition, au plus 0,25 % en poids de la composition, au plus 0,2 % en poids de la composition.

11. Utilisation du système selon l'une quelconque des revendications précédentes, dans laquelle l'enzyme de la première composition représente au moins 7,5 % en poids de la composition, par exemple au moins 6 % en poids de la composition, au moins 5 % en poids de la composition, au moins 4 % en poids de la composition, au moins 3 % en poids de la composition, au moins 2,75 % en poids de la composition, au moins 2,5 % en poids de la composition.

12. Utilisation du système selon l'une quelconque des revendications précédentes, ladite première composition comprenant en outre un agent capable de neutraliser le polymère.

13. Utilisation du système selon la revendication 12, dans laquelle l'agent est la diisopropanolamine.

14. Utilisation du système selon l'une quelconque des revendications précédentes, dans laquelle la première composition est sous la forme d'une crème, d'une pâte, d'un gel ou d'un liquide.

15. Utilisation du système selon la revendication 1, dans laquelle les deux compositions des deux composants du système sont dans des compartiments séparés.

16. Utilisation du système selon la revendication 1, dans laquelle la deuxième composition du deuxième composant du système comprend l'au moins un agent activateur d'enzyme constitué essentiellement d'un solvant.

17. Utilisation du système selon la revendication 1, dans laquelle la deuxième composition du deuxième composant du système comprend l'au moins un agent activateur d'enzyme constitué essentiellement d'eau.

18. Utilisation du système selon la revendication 1, dans laquelle la deuxième composition du deuxième composant du système comprend en outre au moins un agent anti-inflammatoire favorisant la pénétration.

19. Utilisation du système selon la revendication 1, dans laquelle la deuxième composition du deuxième composant du système comprend en outre l'au moins un agent anti-inflammatoire favorisant la pénétration choisi dans le groupe des salicylates.

20. Utilisation du système selon la revendication 1, dans laquelle la deuxième composition du deuxième composant du système comprend en outre au moins un agent conservateur.

21. Utilisation du système selon l'une quelconque des revendications 1 à 8, dans laquelle l'au moins un agent activateur d'enzyme de la deuxième composition du deuxième composant du système représente 95 % en poids du deuxième composant.

22. Utilisation du système selon l'une quelconque des revendications 1 à 8, dans laquelle l'au moins un agent anti-inflammatoire favorisant la pénétration de la deuxième composition du deuxième composant du système représente 2 % en poids de ladite deuxième composition du deuxième composant du système.

23. Utilisation du système selon l'une quelconque des revendications 1 à 8, dans laquelle l'au moins un agent conservateur de la deuxième composition du deuxième composant du système représente 1 % en poids de ladite deuxième composition du deuxième composant du système.
